(19) Europäisches Patentamt
European Patent Office
Office européen des brevets

(11) EP 2 121 143 B1

(12) **EUROPEAN PATENT SPECIFICATION**

(45) Date of publication and mention of the grant of the patent:
**09.09.2015 Bulletin 2015/37**

(21) Application number: **08708978.5**

(22) Date of filing: **14.02.2008**

(51) Int Cl.:
***A61K 8/26*** *(2006.01)* ***A61K 8/34*** *(2006.01)*
***A61K 8/36*** *(2006.01)* ***A61K 8/37*** *(2006.01)*
***A61K 8/39*** *(2006.01)* ***A61Q 19/00*** *(2006.01)*
***A61K 8/89*** *(2006.01)*

(86) International application number:
**PCT/EP2008/051778**

(87) International publication number:
**WO 2008/101852 (28.08.2008 Gazette 2008/35)**

(54) **MALODOR REDUCTION OF COSMETIC PRODUCTS**

GERUCHSREDUZIERUNG FÜR KOSMETIKPRODUKTE

RÉDUCTION DES MAUVAISES ODEURS DE PRODUITS COSMÉTIQUES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **23.02.2007 US 678290**

(43) Date of publication of application:
**25.11.2009 Bulletin 2009/48**

(73) Proprietors:
- **Unilever PLC**
  **London EC4Y 0DY (GB)**
  Designated Contracting States:
  **CY GB IE MT**
- **Unilever N.V.**
  **3013 AL Rotterdam (NL)**
  Designated Contracting States:
  **AT BE BG CH CZ DE DK EE ES FI FR GR HR HU IS IT LI LT LU LV MC NL NO PL PT RO SE SI SK TR**

(72) Inventors:
- **YANG, Lin**
  **Trumbull, Connecticut 06611 (US)**
- **SHAFER, Georgia**
  **Trumbull, Connecticut 06611 (US)**
- **LIPS, Alexander**
  **Parkgate, Cheshire, CH64 6SQ (GB)**

(74) Representative: **Acham, Nicholas Clive et al**
**Unilever Patent Group**
**Colworth House**
**Sharnbrook**
**Bedford**
**MK44 1LQ (GB)**

(56) References cited:

| | |
|---|---|
| **EP-A- 0 306 236** | **EP-A- 1 419 761** |
| **EP-A- 1 702 886** | **WO-A-01/60331** |
| **WO-A-03/045168** | **WO-A-2006/056283** |
| **WO-A-2006/117055** | **DE-A1- 10 311 585** |
| **FR-A- 2 741 533** | **US-A1- 2005 063 928** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 121 143 B1

## Description

The present invention is directed to a skin care composition and a method for reducing malodor in skin care compositions. More particularly, the invention is directed to skin care compositions comprising an insoluble particle that is suitable to adsorb compounds that have been proven to yield offensively unpleasant odors in compositions, like lotions, creams and body washes. The insoluble particle is preferably used with the adsorbable solvent suitable to be adsorbed on to the insoluble particles whereby compositions that contain an insoluble particle and the adsorbable solvent surprisingly show a reduction in malodor, and especially, a reduction in malodor originating from compounds suitable to, for example, hydrogen bond to the insoluble particle and/or be scavenged by the adsorbable solvent which is part of an adsorbable solvent-insoluble particle complex. Furthermore the compositions of the present invention, which comprise an adsorbable solvent-insoluble particle complex, unexpectedly show a reduction in malodor that is greater than the sum of malodor reduction for compositions that only contain insoluble particle and only contain adsorbable solvent.

A wide variety of skin care compositions tend to generate malodors after coming into contact with air, bacteria, skin or combinations of the same for prolonged periods of time. In fact many skin care compositions comprise actives that, for example, oxidize, thereby generating volatile components that result in malodor.

Attempts at reducing malodor in skin care compositions have been made. For example, fragrances have been used in skin care compositions to mask malodors. Use of fragrances, however, is not always desirable since many consumers wish to use skin care compositions that are free of fragrances due to various skin sensitivities and allergies. Also, fragrances within a product tend to have a shorter life than the product itself. Therefore malodor masking may not be achieved during an entire product life.

There is increasing interest to develop skin care compositions that are free of malodor, and especially, skin care compositions that are free of malodor and that are suitable to yield the characteristic benefit they are known to produce. This invention is directed to a skin care composition comprising insoluble particles and preferably an adsorbable solvent. The skin care compositions made according to this invention are surprisingly free of malodor originating from compounds that, for example, are suitable to hydrogen bond to the insoluble particle and/or are scavenged by the adsorbable solvent in an adsorbable solvent-insoluble particle complex.

Efforts have been disclosed for making cosmetic compositions. In WO 93/18130, malodor personal cleansing bars with zeolite are described.

Other efforts have been disclosed for making cosmetic compositions. In US 2006/0135385 A1, toilet bar compositions with pyran odor masking agents are described.

Still other efforts have been disclosed for making consumer product compositions with reduced odor. In EP 0 063 899 A2, fabric conditioning compositions with aluminum chlorohydrate are described.

Further efforts have been disclosed for making cosmetic compositions. In JP 2004290573 A, deodorants having elasticity and flexibility are described whereby the same uses clay as a swelling agent.

In WO 03/045168, an adsorbate of an ester of conjugated linoleic acid and silicate coated with poly ethylene glycol is disclosed.

None of the additional information above describes a skin care composition that has an insoluble particle and adsorbable solvent whereby the composition is free of malodor originating from compounds suitable to, for example, hydrogen bond to the insoluble product and adsorb to the adsorbable solvent in an adsorbable solvent-insoluble particle complex.

## SUMMARY OF THE INVENTION

In a first aspect, the present invention is directed to a method for reducing malodor in a skin care composition according to claim 1.

In a second aspect, the present invention is directed to a malodor-free skin care composition according to claim 2.

Additional aspects of the present invention will more readily become apparent from the description and examples which follow.

Skin, as used herein, is meant to include all skin on the face and body. Skin care composition is meant to mean a composition that may be applied to skin and/or hair as a leave on and/or rinse off composition. Such a skin care composition is not limited with respect to the form it takes and therefore can be, for example, a bar, liquid, gel, stick, roll-on formulation, cream, aerosol or non-aerosol spray, fabric (e.g. non-woven textile) - applied formulation, mousse, lotion, ointment, cosmetic, cosmetic remover, foundation, conditioner or shampoo. The skin care composition is not limited in use and can, for example, lighten, moisturize, clean, nourish, or reduce wrinkles or oil on skin as well as clean, condition or be used to sculpt hair.

Ingredients that can degrade to yield a component with malodor, as used herein, is meant to mean any ingredient that is often used in a topical composition like those that provide a benefit to hair or skin when, for example, being topically applied. Component with a malodor is meant to include, for example, heterocompounds like low molecular

weight ($<C_{10}$) aldehydes and amines that can be found in skin care compositions. Free of malodor or malodor-free is meant to mean free of odor that is offensive and, for example, free of an odor generally produced by aldehydes such as hexanal. Component with malodor and malodor component are meant to be the same. Adsorbable solvent in a adsorbable solvent-insoluble particle complex means a solvent that adsorbs to the insoluble particle often resulting from polar forces that reduce or prevent the adsorbable solvent from mixing with any additional solvent in the skin care composition. The adsorbable solvent, therefore, is the solvent that has the greatest affinity (i.e. greatest adsorbability) for the insoluble particle in comparison to any other solvent in the skin care composition. Solubility parameter distance of the adsorbable solvent as it relates to the component with malodor targeted for scavenging, Ra, may be calculated from the following formula:

$$Ra = (4(\delta_{D1} - \delta_{D2})^2 + (\delta_{p1} - \delta_{p2})^2 + (\delta_{H1} - \delta_{H2})^2)^{1/2}$$

where $\delta_{D1}$ is malodor component dispersion cohesion energy, $\delta_{D2}$ is adsorbable solvent dispersion cohesion energy, $\delta_{p1}$ is malodor component polar cohesion energy, $\delta_{p2}$ is adsorbable solvent dispersion cohesion energy, $\delta_{H1}$ is malodor component hydrogen bonding cohesion energy and $\delta_{H2}$ is adsorbable solvent hydrogen bonding cohesion energy. An additional description of solubility parameter distance may be found in Hansen Solubility Parameters, Hansen, C.M., Chapter 1, CRC Press, 2000, the disclosure of which is incorporated herein by reference. Scavanged, as used herein, means attracted and/or adsorbed to so that an undesirable characteristic, like malodor, can be reduced or eliminated.

## DETAILED DESCRIPTION OF THE INVENTION

The type of ingredients (including active ingredients) that can degrade to yield a component with malodor used in this invention are polyunsaturated fatty acids, wherein the poly unsaturated fatty acid comprises conjugated linoleic acid (CLA). CLA can comprise a group of positional and geometric isomers of linoleic acid in which various configurations of cis and trans double bonds at positions (6,8), (7,9), (8,10), (9,11), (10,12), (11,13), or mixtures thereof are possible. Therefore, many individual isomers and combinations of isomers may be used as the active ingredient (that yields malodor) in this invention.

A preferred CLA suitable for use in the compositions made in accordance with the present invention is the cis 9, trans 11 (hereinafter referred to as c9, t11) isomer. This particular isomer of the free acid has the structure shown below:

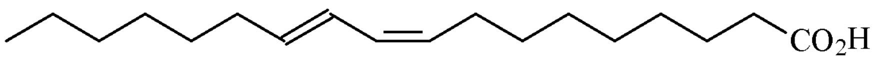

The invention also includes for use as actives derivatives of the free acid (which often comprise conjugated linoleic acid moieties) that can generate a compound with malodor. Preferable derivatives include those derived from substitution of the carboxyl group of the acid, such as esters (e.g. retinyl esters, triglyceride esters, monoglycerides esters diglyceride esters and phosphoesters), amides (e.g. ceramide derivatives), salts (e.g. alkali metal and alkali earth metal salts and ammonium salts); and/or those derived from substitution of the C18 carbon chain, such as alpha and/or beta alkoxy and/or hydroxy derivatives.

In the case of triglyceride ester derivatives, all positional isomers of CLA substituents on the glycerol backbone are included. The triglycerides should contain at least one CLA moiety. For example, of the three esterifiable positions on the glycerol backbone, the 1 and 2 positions may be esterified with CLA and by another lipid at position 3 or as an alternative, the glycerol backbone could be esterified by CLA at the 1 and 3 positions with another lipid at position 2.

"CLA moieties" are also included. "CLA moieties" refer to CLA fatty acyl portion(s) of a CLA derivative.

By "c9, t11 isomer enriched CLA" is meant that at least about 30% by weight of the total CLA and/or CLA moieties present in the composition is in the form of the cis 9, trans 11 isomer. Preferably, at least about 35%, and most preferably, at least 40% to 90% by weight of the total CLA and/or CLA moieties present in the composition, are in the form of the c9, t11 isomer, including all ranges subsumed therein.

However in one particular preferred embodiment, cis 9, trans 11 isomer and trans 10, cis 12 isomer (or any derivatives thereof) are present as the active at a weight ratio from 40:60 to 60:40, and preferably, at a weight ratio from 45:55 to 55:45, including all ratios subsumed therein. CLA type products suitable for use in this invention are available from suppliers like Stepan under the name Neobee® and Loders Croklaan under the name Clarinol™.

The CLA and/or derivatives thereof comprising CLA moieties according to the present invention may be prepared, for example, according to the method disclosed in WO 97/18320, the disclosure of which is incorporated herein

by reference.

The CLA to be employed in accordance with the present invention is typically present in the skin care composition in an effective amount. Normally, the total amount of the ingredient that can degrade to yield a component with malodor is present in an amount from 0.00001% to 50% by weight of the composition. More preferably, the amount is from 0.01% to 10%, and most preferably, from 0.1 % to 5% by weight of the composition, including all ranges subsumed therein.

Another ingredient that can degrade to yield a component with a malodor and that is suitable for use in this invention is a monoenoic fatty acid (i.e. monounsaturated fatty acid) like cis-4-decenoic, cis-9-decenoic, cis-5-lauroleic, cis-4-dodecenoic, cis-9-tetradecenoic, cis-5-teradecenoic, cis-4-tetradecenoic, cis-9-hexadecenoic, cis-6-octadecenoic, cis-9-octadecenoic, tr-9-octadecenoic, cis-11-octadecenoic, cis-9-eicosenoic, cis-11-eicosenoic, cis-11-docosenoic, cis-13-docosenoic, cis-15-tetracosenoic acid, derivatives thereof or mixtures thereof.

The preferred monoenoic fatty acid suitable for use in this invention is cis-6-octadecenoic acid (i.e. petroselinic acid) whereby the same is used in combination with CLA.

If desired for use, the amount of monoenoic acid employed in the skin care composition of this invention is often from 0.005 to 35%, and preferably, from 0.01 to 25%, and most preferably, from 0.5 to 6% by weight, including all ranges subsumed therein.

As to component with malodor originally formulated in the skin care composition of this invention, such a component is not limited and often is one which comprises a heteroatom, and especially, nitrogen.

The only limitations with respect to the adsorbable solvent that may be used in this invention in that the same is suitable for use in a skin care composition, is not classified as a solvent with malodor (e.g. does not possess an aroma that is similar to hexanal) and has a solubility parameter distance as it relates to the component with a malodor targeted for scavaging of less than 20. Regarding the adsorbable solvent, the same typically has an Ra of less than 20, and preferably, less than 18, and most preferably, from 1 to 15, including all ranges subsumed therein. Ra was determined using Molecular Modelling Pro (MMP) software available from ChemSW® (420-F Executive Court North, Fairfield, CA 94585, US).

The adsorbable solvent in this invention is dipropylene glycol.

The the adsorbable solvent makes up from 0.01 to 35%, and preferably, from 0.05 to 20%, and most preferably, from 0.1 to 5% by weight of the skin care composition, including all ranges subsumed therein. Often, the amount of adsorbable solvent employed in the skin care composition of this invention is from 0.1 to 25%, and preferably, from 1 to 12%, and most preferably, from 1 to 5% by weight, based on total weight of the skin care composition and including all ranges subsumed therein.

The insoluble particle has a surface area from 75 to 3500 $m^2/g$. The surface area referred to is the specific surface area (SSA) which is defined as the total area of the particles divided by the total weight of the particles ($m^2/g$). Values are obtained from a dry powder at room temperature using a Malvern Mastersizer 2000 assuming the particles are spherical and non-porous.

Preferred synthetic layered silicates include those prepared from salts of sodium, magnesium and lithium and sold under the name Laponite® (sodium, lithium and magnesium silicate made available by Southern Clay Products Incorporated).

Illustrative fumed silicas that may be used include those sold under the name Aerosil (available from Degussa AG), Cab-o-sil (available from Cabot), mixtures thereof and the like.

The amount of insoluble particle used in the skin care composition of the present invention is from 0.1 to 10, and preferably, from 0.1 to 8, and most preferably, from 0.2 to 6 weight percent, based on total weight of the skin care composition, including all ranges subsumed therein.

Water is typically the solvent (i.e. the solvent that is used in addition to the adsorbable solvent) employed in this invention wherein water will make up the balance of the skin care composition.

Emollient materials may serve as cosmetically acceptable carriers for the skin care composition of this invention. These may be in the form of silicone oils, natural or synthetic esters and hydrocarbons. Amounts of the emollients may range anywhere from 0.1 to 95%, preferably between 1 and 50% by weight of the composition.

Silicone oils may be divided into the volatile and non-volatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms.

Non-volatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from $5 \times 10^{-6}$ to 0.1 $m^2/s$ at 25°C. Among the preferred non-volatile emollients useful in the present compositions are the polydimethyl siloxanes having viscosities from $1 \times 10^{-5}$ to $4 \times 10^{-4}$ $m^2/s$ at 25°C.

Another class of non-volatile silicones are emulsifying and non-emulsifying silicone elastomers. Representative of this category is dimethicone/vinyl dimethicone crosspolymer available as Dow Corning 9040, General Electric SFE 839, and Shin-Etsu KSG-18. Silicone waxes such as Silwax WS-L (dimethicone copolyol laurate) may also be useful.

Among the ester emollients are:

a) Alkyl esters of saturated fatty acids having 10 to 24 carbon atoms. Examples thereof include behenyl neopentanoate, isononyl isonanonoate, isopropyl myristate and octyl stearate.
b) Ether-esters such as fatty acid esters of ethoxylated saturated fatty alcohols.
c) Polyhydric alcohol esters. Ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of $C_1$-$C_{30}$ alcohols.
d) Wax esters such as beeswax, spermaceti wax and tribehenin wax.
e) Sugar ester of fatty acids such as sucrose polybehenate and sucrose polycottonseedate.

Natural ester emollients principally are based upon mono-, di- and tri- glycerides. Representative glycerides include sunflower seed oil, cottonseed oil, borage oil, borage seed oil, primrose oil, castor and hydrogenated castor oils, rice bran oil, soybean oil, olive oil, safflower oil, shea butter, jojoba oil and combinations thereof. Animal derived emollients are represented by lanolin oil and lanolin derivatives. Amounts of the natural esters may range from 0.1 to 20% by weight of the compositions.

Hydrocarbons which are suitable cosmetically acceptable carriers include petrolatum, mineral oil, $C_{11}$-$C_{13}$ isoparaffins, polybutenes, and especially isohexadecane, available commercially as Permethyl 101A from Presperse Incorporated.

Fatty acids having from 10 to 30 carbon atoms may also be suitable as cosmetically acceptable carriers. Illustrative of this category are pelargonic, lauric, myristic, palmitic, stearic, isostearic, oleic, linoleic, linolenic, hydroxystearic and behenic acids.

Fatty alcohols having from 10 to 30 carbon atoms are another useful category of cosmetically acceptable carrier. Illustrative of this category are stearyl alcohol, lauryl alcohol, myristyl alcohol, oleyl alcohol and cetyl alcohol.

Thickeners can be utilized as part of the cosmetically acceptable carrier of compositions according to the present invention. Typical thickeners include crosslinked acrylates (e.g. Carbopol 982®), hydrophobically-modified acrylates (e.g. Carbopol 1382®), polyacrylamides (e.g. Sepigel 305®), acryloylmethylpropane sulfonic acid/salt polymers and copolymers (e.g. Aristoflex HMB® and AVC®), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Inorganics may also be utilized as thickeners, particularly clays such as bentonites and hectorites, fumed silicas, talc, calcium carbonate and silicates such as magnesium aluminum silicate (Veegum®). Amounts of the thickener may range from 0.0001 to 10%, usually from 0.001 to 1%, optimally from 0.01 to 0.5% by weight of the composition.

Adjunct humectants may be employed in the present invention. These are generally polyhydric alcohol-type materials. Typical polyhydric alcohols include glycerol, propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of adjunct humectant may range anywhere from 0.5 to 50%, preferably between 1 and 15% by weight of the composition.

Surfactants may also be present in compositions of the present invention. The total concentration of the surfactant when present may range from 0.1 to 90%, preferably from 1 to 40%, optimally from 1 to 20% by weight of the composition, and being highly dependent upon the type of personal care product. The surfactant may be selected from the group consisting of anionic, nonionic, cationic and amphoteric actives. Particularly preferred nonionic surfactants are those with a $C_{10}$-$C_{20}$ fatty alcohol or acid hydrophobe condensed with from 2 to 100 moles of ethylene oxide or propylene oxide per mole of hydrophobe; $C_2$-$C_{10}$ alkyl phenols condensed with from 2 to 20 moles of alkylene oxide; mono- and di-fatty acid esters of ethylene glycol; fatty acid monoglyceride; sorbitan, mono- and di- $C_8$-$C_{20}$ fatty acids; polyoxyethylene sorbitan; as well as combinations thereof. Alkyl polyglycosides and saccharide fatty amides (e.g. methyl gluconamides) and trialkylamine oxides are also suitable nonionic surfactants.

Preferred anionic surfactants include soap, alkyl ether sulfates and sulfonates, alkyl sulfates and sulfonates, alkylbenzene sulfonates, alkyl and dialkyl sulfosuccinates, $C_8$-$C_{20}$ acyl isethionates, $C_8$-$C_{20}$ alkyl ether phosphates, $C_8$-$C_{20}$ sarcosinates, $C_8$-$C_{20}$ acyl lactylates, sulfoacetates and combinations thereof. An often most preferred anionic surfactant is sodium dodecyl sulfate (SDS).

Useful amphoteric surfactants include cocoamidopropyl betaine, $C_{12}$-$C_{20}$ trialkyl betaines, sodium lauroamphoacetate and sodium laurodiamphoacetate.

Sunscreen agents may also be included in compositions of the present invention. Particularly preferred are such materials as ethylhexyl p-methoxycinnamate (available as Parsol MCX®), Avobenzene (available as Parsol 1789®) and benzophenone-3 (also known as Oxybenzone). Inorganic sunscreen actives may be employed such as microfine titanium dioxide and zinc oxide. Amounts of the sunscreen agents when present may generally range from 0.1 to 30%, preferably from 2 to 20%, optimally from 4 to 10% by weight of the composition.

Certain skin care compositions of the present invention ordinarily will contain astringent actives. Examples include aluminum chloride, aluminum chlorhydrex, aluminum-zirconium chlorhydrex glycine, aluminum sulfate, zinc sulfate, zirconium and aluminum chlorohydroglycinate, zirconium hydroxychloride, zirconium and aluminum lactate, zinc phenolsulfonate and combinations thereof. Amounts of the astringents may range anywhere from 0.5 to 50% by weight of the composition.

Preservatives can desirably be incorporated into the skin care compositions of this invention to protect against the growth of potentially harmful microorganisms. Particularly preferred preservatives are phenoxyethanol, methyl paraben, propyl paraben, imidazolidinyl urea, dimethyloldimethylhydantoin, ethylenediaminetetraacetic acid salts (EDTA), sodium dehydroacetate, methylchloroisothiazolinone, methylisothiazolinone, iodopropynbutylcarbamate and benzyl alcohol. The preservatives should be selected having regard for the use of the composition and possible incompatibilities between the preservatives and other ingredients. Preservatives are preferably employed in amounts ranging from 0.01% to 2% by weight of the composition.

Compositions of the present invention may include vitamins. Illustrative vitamins are vitamin A (retinol), vitamin $B_2$, vitamin $B_3$ (niacinamide), vitamin $B_6$, vitamin C, vitamin E, folic acid and biotin. Derivatives of the vitamins may also be employed. For instance, vitamin C derivatives include ascorbyl tetraisopalmitate, magnesium ascorbyl phosphate and ascorbyl glycoside. Derivatives of vitamin E include tocopheryl acetate, tocopheryl palmitate and tocopheryl linoleate. DL-panthenol and derivatives may also be employed. For the purposes of this invention, vitamins where present are not considered as unsaturated materials. The total amount of vitamins when present in compositions according to the present invention may range from 0.001 to 10%, preferably from 0.01% to 1%, optimally from 0.1 to 0.5% by weight of the composition.

Another type of useful substance can be that of an enzyme such as amylases, oxidases, proteases, lipases and combinations. Particularly preferred is superoxide dismutase (commercially available as Biocell SOD from the Brooks Company, USA).

Skin lightening compounds may be included in the compositions of the invention. Illustrative substances are placental extract, lactic acid, niacinamide, arbutin, kojic acid, ferulic acid, resorcinol and derivatives including 4-substituted resorcinols and combinations thereof. Amounts of these agents may range from 0.1 to 10%, preferably from 0.5 to 2% by weight of the composition.

Desquamation promoters may be present. Illustrative are the alpha-hydroxycarboxylic acids and beta-hydroxycarboxylic acids. The term "acid" is meant to include not only the free acid but also salts and $C_1$-$C_{30}$ alkyl or aryl esters thereof and lactones generated from removal of water to form cyclic or linear lactone structures. Representative acids are glycolic, lactic and malic acids. Salicylic acid is representative of the beta-hydroxycarboxylic acids. Amounts of these materials when present may range from 0.01 to 15% by weight of the composition.

A variety of herbal extracts may optionally be included in compositions of this invention. The extracts may either be water soluble or water-insoluble carried in a solvent which respectively is hydrophilic or hydrophobic. Water and ethanol are the preferred extract solvents. Illustrative extracts include those from green tea, chamomile, licorice, aloe vera, grape seed, citrus unshui, willow bark, sage, thyme and rosemary.

Also included may be such materials as lipoic acid, retinoxytrimethylsilane (available from Clariant Corporation. under the Silcare 1 M-75 trademark), dehydroepiandrosterone (DHEA) and combinations thereof. Ceramides (including Ceramide 1, Ceramide 3, Ceramide 3B and Ceramide 6) as well as pseudoceramides may also be useful. Amounts of these materials may range from 0.000001 to 10%, preferably from 0.0001 to 1 % by weight of the composition.

Colorants, opacifiers and abrasives may also be included in compositions of the present invention. Each of these substances may range from 0.05 to 5%, preferably between 0.1 and 3% by weight of the composition.

The compositions of the present invention can also be, optionally, incorporated into an insoluble substrate for application to the skin such as in the form of a treated wipe.

A wide variety of packaging can be employed to store and deliver the skin care compositions. Packaging is often dependent upon the type of personal care end-use. For instance, leave-on skin lotions and creams, shampoos, conditioners and shower gels generally employ plastic containers with an opening at a dispensing end covered by a closure. Typical closures are screw-caps, non-aerosol pumps and flip-top hinged lids. Packaging for antiperspirants, deodorants and depilatories may involve a container with a roll-on ball on a dispensing end. Alternatively these types of skin care compositions may be delivered in a stick composition formulation in a container with propel-repel mechanism where the stick moves on a platform towards a dispensing orifice. Metallic cans pressurized by a propellant and having a spray nozzle serve as packaging for antiperspirants, shave creams and other personal care products. Toilette bars may have packaging constituted by a cellulosic or plastic wrapper or within a cardboard box or even encompassed by

a shrink wrap plastic film. All of the aforementioned are considered packaging within the context of the present invention.

The examples are provided to facilitate an understanding of the present invention and they are not meant to limit the scope of the claims.

Example 1

Emulsions (i.e. skincare compositions) comprising CLA {50% cis 9, trans 11 and 50% trans 10, cis 12} were prepared by mixing CLA, sodium dodecyl sulfate (about 0.6%) and water. The resulting mixture was stirred on a stirring plate for about 20 minutes in order to dissolve the SDS into solution. The SDS solution with dispersed CLA was sonicated for about 2 minutes using an Ulbra Cell™ sonicator to yield stable emulsions. A first stable emulsion was completed by adding 2% DPG, a second was completed by adding 2.5% clay (Laponite® grade XLG) and a third was completed by adding 2% DPG and 2.5% clay (Laponite® grade XLG). All completed emulsions were subjected to an additional two minutes of sonication. 3% CLA was present in each emulsion, water was added to balance and all percents are percents by weight based on total weight of the emulsion.

Example 2

Headspace analysis was performed on the CLA emulsions prepared in example 1. Solid phase microextraction (SPME) - gas chromatography (GC) 6890 / mass spectrometry (MS) 5973 / flame ionization detector (FID) from Agilent was used to identify the chemical composition of the vapor (i.e. aldehydes resulting from, for example, the oxidation of CLA) over aged (i.e. greater than 3 months old and stored at 60°C) emulsions. One gram of each of the above-described emulsions was filled in 20ml GC headspace sampling vials sealed with caps and septum. The GC column used was an HP-5MS column from Agilent (inner diameter 0.25mm, length 30m, stationary phase thickness 0.25um). GC conditions were such that the injector was in the splitless mode with helium gas as the carrier gas. The injection port was heated to 250°C, with purge flow at split vent 50ml/min for 2 minutes. The column was set at a constant flow mode of 1.3 ml/min. Oven temperature ramp was held at 75°C for 2 minutes, and increased at a rate of 6°C/min to 100°C, 1.5°C/min to 150°C, 3°C/min to 190°C, 30°C/min to 300°C and held for two minutes. MS conditions were such that solvent delay was for 0.5 minutes and the scan started from low mass 35 to high mass 300. The autosampler conditions were such that the samples were incubated at 35°C for 25 minutes with no agitation. SPME fiber was inserted into the sample headspace for a 5 minutes extraction and subsequently injected into the injector for a 15 minute desorption.

The results in the table below demonstrate that the use of an insoluble particle and adsorbable solvent unexpectedly show a reduction in malodor that is greater than the sum of malodor reduction for compositions that only contain an insoluble particle and only contain adsorbable solvent.

TABLE

| Component with Malodor | Insoluble Particle and/or Adsorbable Solvent | Malodor in Headspace Relative Concentration |
|---|---|---|
| Butanal | Control | 1 |
| Butanal | DPG | 0.88 |
| Butanal | Laponite | 0.1 |
| Butanal | Laponite + DPG | 0.05 |
| Pentanal | Control | 1 |
| Pentanal | DPG | 0.93 |
| Pentanal | Laponite | 0.22 |
| Pentanal | Laponite + DPG | 0.05 |
| N-hexanal | Control | 1.0 |
| N-hexanal | DPG | 0.96 |
| N-hexanal | Laponite | 0.3 |
| N-hexanal | Laponite + DPG | 0.05 |
| Heptanal | Control | 1.0 |
| Heptanal | DPG | 0.94 |

(continued)

| Component with Malodor | Insoluble Particle and/or Adsorbable Solvent | Malodor in Headspace Relative Concentration |
|---|---|---|
| Heptanal | Laponite | 0.35 |
| Heptanal | Laponite + DPG | 0.1 |
| 2-Octenal | Control | 1 |
| 2-Octenal | DPG | 0.9 |
| 2-Octenal | Laponite | 0.3 |
| 2-Octenal | Laponite + DPG | 0.06 |
| Nonenal | Control | 1 |
| Nonenal | DPG | 0.9 |
| Nonenal | Laponite | 0.22 |
| Nonenal | Laponite + DPG | 0.05 |

**Claims**

**1.** A method for reducing malodor in a skin care composition comprising the steps of:

(a) formulating the skin care composition with an ingredient that can degrade to yield a component with a malodor; and
(b) including in the skin care composition an insoluble particle and an adsorbable solvent, the component with a malodor being one suitable to hydrogen bond with the insoluble particle and/or be scavanged by the adsorbable solvent in an adsorbable solvent-insoluble particle complex

wherein the insoluble particle has a surface area from 75 to 3500 $m^2/g$,
wherein the ingredient that can degrade to yield a component with malodor is a polyunsaturated fatty acid,
wherein the polyunsaturated fatty acid comprises conjugated linoleic acid,
wherein the insoluble particle comprises sodium, lithium and magnesium silicate,
wherein the adsorbable solvent is dipropylene glycol,
wherein the term "adsorbable solvent" means a solvent that adsorbs to the insoluble particle reducing or preventing the adsorable solvent from mixing with any additional solvent in the skin care composition, and
wherein the skin care composition comprises from 0.01 to 35% by weight adsorbable solvent and from 0.1 to 10% by weight insoluble particle.

**2.** A skin care composition comprising:

(a) carrier;
(b) insoluble particle;
(c) adsorbable solvent; and
(d) a component with a malodor,

wherein the component with a malodor is one which is suitable to hydrogen bond with the insoluble particle and/or be scavanged by the adsorbable solvent in an adsorbable solvent-insoluble particle complex further wherein the insoluble particle has a surface area from 75 to 3500 $m^2/g$,
wherein the skin care composition comprises from 0.01 to 35% by weight adsorbable solvent and from 0.1 to 10% by weight insoluble particle.,
wherein the insoluble particle comprises sodium, lithium and magnesium silicate,
wherein the adsorbable solvent is dipropylene glycol,
wherein the component with a malodor is a degradation product of a polyunsaturated acid,
wherein the polyunsaturated acid comprises conjugated linoleic acid, and wherein the term "adsorbable solvent" means a solvent that adsorbs to the insoluble particle reducing or preventing the adsorable solvent from mixing with

any additional solvent in the skin care composition.

## Patentansprüche

1. Verfahren zur Geruchsreduzierung in einer Hautpflegezusammensetzung, umfassend die Schritte:

   (a) Formulieren der Hautpflegezusammensetzung mit einem Inhaltsstoff, der zerfallen kann, um einen Bestandteil mit einem üblen Geruch zu ergeben; und
   (b) Einbeziehen eines unlöslichen Teilchens und eines adsorbierbaren Lösungsmittels in die Hautpflegezusammensetzung, wobei der Bestandteil mit einem üblen Geruch einer ist, der geeignet für Wasserstoffbrückenbindung mit dem unlöslichen Teilchen ist und/oder dazu, durch das adsorbierbare Lösungsmittel in einem Komplex aus adsorbierbarem Lösungsmittel und unlöslichem Teilchen abgefangen zu werden,

   wobei das unlösliche Teilchen eine spezifische Oberfläche von 75 bis 3500 $m^2/g$ hat, wobei der Inhaltsstoff, der zerfallen kann, um einen Bestandteil mit einem üblen Geruch zu ergeben, eine mehrfach ungesättigte Fettsäure ist,
   wobei die mehrfach ungesättigte Fettsäure konjugierte Linolsäure umfasst,
   wobei das unlösliche Teilchen Natrium-, Lithium- und Magnesiumsilicat umfasst,
   wobei das adsorbierbare Lösungsmittel Dipropylenglycol ist,
   wobei der Ausdruck "adsorbierbares Lösungsmittel" ein Lösungsmittel bedeutet, welches an dem unlöslichen Teilchen adsorbiert wird, wodurch das adsorbierbare Lösungsmittel an der Mischung mit irgendeinem zusätzlichen Lösungsmittel in der Hautpflegezusammensetzung gehindert oder diese verringert wird, und
   wobei die Hautpflegezusammensetzung 0,01 bis 35 Gew.-% adsorbierbares Lösungsmittel und 0,1 bis 10 Gew.-% unlösliches Teilchen umfasst.

2. Hautpflegezusammensetzung umfassend:

   (a) Träger;
   (b) unlösliches Teilchen
   (c) adsorbierbares Lösungsmittel; und
   (d) einen Bestandteil mit einem üblen Geruch,

   wobei der Bestandteil mit einem üblen Geruch einer ist, der geeignet für Wasserstoffbrückenbindung mit dem unlöslichen Teilchen ist und/oder dazu, durch das adsorbierbare Lösungsmittel in einem Komplex aus adsorbierbarem Lösungsmittel und unlöslichem Teilchen abgefangen zu werden, wobei ferner das unlösliche Teilchen eine spezifische Oberfläche von 75 bis 3500 $m^2/g$ hat, wobei die Hautpflegezusammensetzung 0,01 bis 35 Gew.-% adsorbierbares Lösungsmittel und 0,1 bis 10 Gew.-% unlösliches Teilchen umfasst,
   wobei das unlösliche Teilchen Natrium-, Lithium- und Magnesiumsilicat umfasst,
   wobei das adsorbierbare Lösungsmittel Dipropylenglycol ist,
   wobei der Bestandteil mit einem üblen Geruch ein Abbauprodukt einer mehrfach ungesättigten Fettsäure ist,
   wobei die mehrfach ungesättigte Fettsäure konjugierte Linolsäure umfasst, und
   wobei der Ausdruck "adsorbierbares Lösungsmittel" ein Lösungsmittel bedeutet, welches an dem unlöslichen Teilchen adsorbiert wird, wodurch das adsorbierbare Lösungsmittel an der Mischung mit irgendeinem zusätzlichen Lösungsmittel in der Hautpflegezusammensetzung gehindert oder diese verringert wird.

## Revendications

1. Procédé de réduction d’une mauvaise odeur dans une composition pour le soin de la peau comprenant les étapes :

   (a) de formulation de la composition pour le soin de la peau avec un ingrédient qui peut se décomposer pour produire un constituant avec une mauvaise odeur ; et
   (b) incluant dans la composition pour le soin de la peau une particule insoluble et un solvant adsorbable, le constituant avec une mauvaise odeur étant approprié à une liaison hydrogène avec la particule insoluble et/ou à être fixé par le solvant absorbable dans un complexe de solvant adsorbable-particule insoluble,

   dans lequel la particule insoluble présente une surface spécifique de 75 à 3 500 $m^2/g$,
   dans lequel l’ingrédient qui peut se décomposer pour produire un constituant avec une mauvaise odeur est un acide

gras polyinsaturé,
dans lequel l'acide gras polyinsaturé comprend de l'acide linoléique conjugué,
dans lequel la particule insoluble comprend du silicate de sodium, lithium et magnésium,
dans lequel le solvant adsorbable est le dipropylèneglycol,
dans lequel le terme "solvant adsorbable" indique un solvant qui adsorbe par rapport à la particule insoluble réduisant ou évitant que le solvant adsorbable se mélange avec tout solvant supplémentaire dans la composition pour le soin de la peau, et
dans lequel la composition pour le soin de la peau comprend de 0,01 à 35 % en poids de solvant adsorbable et de 0,1 à 10 % en poids de particule insoluble.

**2.** Composition pour le soin de la peau comprenant :

(a) un support ;
(b) une particule insoluble ;
(c) un solvant adsorbable ; et
(d) un constituant avec une mauvaise odeur,

dans laquelle le constituant avec une mauvaise odeur est approprié à une liaison hydrogène avec la particule insoluble et/ou à être fixé par le solvant adsorbable dans un complexe de solvant adsorbable-particule insoluble dans laquelle de plus la particule insoluble présente une surface spécifique de 75 à 3 500 $m^2/g$,
dans laquelle la composition pour le soin de la peau comprend de 0,01 à 35 % en poids de solvant adsorbable et de 0,1 à 10 % en poids de particule insoluble,
dans laquelle la particule insoluble comprend du silicate de sodium, lithium et magnésium,
dans laquelle le solvant adsorbable est le dipropylèneglycol,
dans laquelle le constituant avec une mauvaise odeur est un produit de décomposition d'un acide polyinsaturé,
dans laquelle l'acide polyinsaturé comprend de l'acide linoléique conjugué, et
dans laquelle le terme "solvant adsorbable" indique un solvant qui absorbe par rapport à la particule insoluble réduisant ou évitant que le colorant adsorbable se mélange avec tout solvant supplémentaire dans la composition pour le soin de la peau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9318130 A
- US 20060135385 A1
- EP 0063899 A2
- JP 2004290573 A
- WO 03045168 A
- WO 9718320 A


### Non-patent literature cited in the description


- **HANSEN, C.M.** Hansen Solubility Parameters. CRC Press, 2000